# EUROPEAN PATENT SPECIFICATION

(11) **EP 1 607 096 B1**
(45) Date of publication and mention of the grant of the patent: **08.08.2007**
(21) Application number: 05010240.9
(22) Date of filing: 11.05.2005
(51) Int. Cl.: A61K 35/74, A23L 1/30, A23L 1/305, A23L 1/0532

(54) **Nutraceutical composition containing milk enzymes useful as synbiotic**
Nutrazeutische Zusammensetzungen enthaltend Milchenzyme als Symbiose
Composition Nutraceutique contenant des enzymes de lait utiles comme symbiotique

(30) Priority: 18.06.2004 IT mi20041232
(43) Date of publication of application: 21.12.2005
(73) Proprietor: Dietetics Pharma S.r.l., 20138 Milano (IT)
(72) Inventor: Angellotti, Carlo, 20129 Milano (IT)
(74) Representative: Savi, Alberto

(56) References cited:
- EP-A- 1 407 678
- EP-A- 1 417 895
- WO-A-01/15714
- WO-A-02/091833
- WO-A-03/041512
- DE-U1- 20 202 562
- US-A1- 2002 001 575
- US-B1- 6 203 797

## Description

This invention relates to a nutraceutical composition containing a large number of live milk enzymes (human and coated) with aloe and carrageenans, in combination with suitable prebiotic substances, which consequently possesses synbiotic activity.

The intestinal microbial flora of individuals of all ages is represented by a myriad of germs (several hundred billion per gram) belonging to over three hundred different species from different taxonomic groups, which normally inhabit the colon and perform multiple metabolic activities that influence the host's state of health. In particular, during the evolution of the human race, an important, delicate balance of mutual advantage developed between man and microflora; however, this balance risks being prejudiced by sudden changes.

Under conditions of mental, physical, dietary or environmental stress, or as a result of drug use, the microflora becomes unbalanced (dysbiosis), and this renders the body liable to attack by pathogens.

In such cases the balance of the microflora and the associated metabolic activity can and should be regulated by suitable measures, including medical, and very often dietary measures, and the use of prebiotics or probiotics is a very helpful corrective measure in such cases.

Prebiotics are non-digestible food ingredients which beneficially influence the host by stimulating the growth and/or activity of one or a limited number of bacterial species resident in the colon, thus improving the health of the host (Gibson et al., "Dietary modulation of the human colonic microbiota: introducing the concept of prebiotics." J. Nutrition 125:167-176 (1995)). Some useful prebiotics are nondigestible oligosaccharides (NOS = nondigestible oligosaccharides), which have a fibre effect and promote the proliferation of bacteria, especially bifidobacteria, in the colon; fructooligosaccharides (FOS = fructooligosaccharides), galactooligosaccharides (GOS), transgalactooligosaccharides (TOS) and arabino galacto oligosaccharides.

Probiotics are diet supplements consisting of live microbes, which improve the health of the individual due to their favourable influence on the intestinal microbiological balances (Salminen et al., "Functional food science and gastrointestinal physiology and function" Brit. J. Nutr. 80 (suppl.): S147-S171(1998). The most suitable probiotics are lactobacilli, bifidobacteria and some streptococci, which must compete with and act as antagonists to undesirable aboriginal intestinal flora, and be able to adhere to the intestinal mucosa.

The attention of researchers in this field has long focused on investigating the effects of a combination of probiotics and prebiotics, called "synbiotics". The synbiotic activity of these new foods is to selectively stimulate the growth and/or metabolism of microbial groups useful to the health of the host, at the same time increasing survival and colonisation by the probiotic micro-organisms present in the product (Andersson et al. "Health effects of probiotics and prebiotics: A literature review on human studies" Scand. J. Nutr. 45:58-75 (2001)).

US 6203797 describes a synbiotic composition comprising probiotics, prebiotics with aloe vera and vitamin B for improving intestinal health.

It has been known for some time that milk enzymes can be used to reduce or eliminate disorders caused by lactose intolerance and intestinal infections, leading to a consequent reduction in the duration of infectious diarrhoea.

Milk enzymes belong to two categories: homolactic and heterolactic. Both are able to use lactose, a disaccharide sugar constituting the milk of the mammals, and oligosaccharides, both of which are subject to cleavage by the constituting or induced enzymes.

Lactase (beta-galactosidase) is the fundamental enzyme that performs hydrolytic cleavage of lactose with a fermentation process controlled by enzymes which act as catalysts, but remain quantitatively present even at the end of the process.

Milk enzymes are the most effective way of advantageously colonising the intestine, not only because they are able to produce vitamins, but also because they compete with any pathogenic germs present, thus constituting an important defence in the battle between the body and pathogenic germs. By means of epithelial adherence, milk enzymes also form a defensive barrier against the unwanted proliferation of some micro-organisms, such as *Escherichia coli, Shigella flexneri, Clostridium difficile,* etc.

A nutraceutical composition with synbiotic activity, having a large number of milk enzymes which are alive after the gastric barrier and survive in the intestinal ecosystem by adhering to the intestinal mucosa, would therefore be required to increase microbial colonisation and aid the host that receives them.

The Applicant has now surprisingly discovered a synbiotic nutraceutical composition which possesses said characteristics. It consists of micro-organisms suitably chosen from among homo- and heterolactic milk enzymes which are able to colonise the transverse colon tract of the gastrointestinal apparatus of the human body, proliferating exponentially until there are tens of billions of germs per gram. Said milk enzymes release biodynamic metabolites of considerable efficacy for the internal structures of the body into their habitat; they include, for example, various coenzymes and enzymes such as beta-galactosidase, nitroreductase, oxypropylmethylcellulase, cellobiase, etc.

In particular, the nutraceutical composition to which this invention relates consists of at least 12 strains of lyophilised double-coated live human milk enzymes and at least one sporified strain of live milk enzymes.

The strains of live and sporified milk enzymes are normally supplied to the Applicant by the Harmonium company; in particular they are present in the quantity of 320 billion/g of double-coated lyophilised human milk enzymes and 200 billion/g of sporified milk enzymes.

To minimise mortality during the gastric transit of said enzymes, the composition according to the invention contains suitable quantities of aloe and carrageenans.

According to a preferred aspect of this invention, 5 to 10 mg of aloe and 15 to 30 mg of carrageenans are present.

It is known that the crucial passage of a composition containing milk enzymes starts at the pit of the stomach, where the pH has a value of approximately 4.2, subsequently reaches the greatest peak of 1.5 in the duodenum, then rises through the small intestine and the intestinal villi at a pH ranging between 4.8 and 5.5, where it gradually begins to dissolve and release living and viable cells.

The gradual dissolution of the composition according to the present invention causes triggers the action of the aloe and carrageenans, which form a protective thickening that protects the milk enzymes, reduces the rate of transit on the villi of the enzymes which are still viable after passing the gastric barrier, and increases the possibility of new colony formation.

Aloe vera is a tropical or sub-tropical plant from the Liliaceae family, whose leaves contain a clear, sticky gel traditionally used as a topical antiinflammatory for irritated skin, while the carrageenans are polysaccharides extracted mainly from seaweed, which are used as thickeners in foodstuffs. In particular, iota carrageenan is used in this invention.

Moreover, the sporified transit strains provided in this invention, such as *Lactobacillus sporogenes,* produce lactic acid and acetic acid, thus combating the growth of pathogenic bacteria and creating the ideal environment for colonisation by resident milk enzymes.

The composition to which this invention relates may also contain glucans, which are a concentrate of the lysed cell walls of *Saccharomyces cerevisiae,* and have a protective action on the intestinal mucosa.

It is known that probiotics (milk enzymes), like all micro-organisms, require nourishment to live. The ideal nutrients to activate their fermentation metabolism are sugars, which are not present in the first tract of the small intestine as they are entirely hydrolysed by the digestive enzymes and absorbed by the intestinal villi. Consequently, even if milk enzymes reach the colon alive, they are unable to compete with the pathogenic bacterial flora.

However, the composition to which this invention relates contains a fibre which reaches the colon intact, providing nourishment for probiotics and consequently enabling them to produce antimicrobial substances (lactic acid, acetic acid, butyric acid, etc.) which inhibit all activity harmful to the body.

In particular, this composition uses the fibres of the larch tree (arabino galactooligosaccharide). This fibre is commonly marketed under the name FIBER-AID® AG.

The composition to which this invention relates may contain, in addition to the combination according to the present invention and any suitable vehicles and/or excipients, even other components or active constituents which promote the growth of the micro-organisms in the combination, such as prebiotic agents like inulin, fructo-oligosaccharides or vitamins, and compounds useful for the purpose of the composition. Excipients and vehicles useful for the composition according to the invention are known to the prior art, and are described, for example, in "Remington's Pharmaceutical Sciences Handbook, Mack Pub., N.Y., USA".

The composition according to the invention can be administered one or more times a day, as required, depending on the patient's health, weight and age.

According to a preferred embodiment of this invention, the composition is administered orally. For oral administration, the composition according to the invention is preferably formulated in a solid phase and a solvent phase in suitable dosage forms, such as ampoules and the like.

The composition according to the invention may contain, in both the solid phase and the solvent phase, suitable vehicles and/or excipients such as a vitamin complex, silica or compounds which can be useful for the final indication of the composition.

According to a preferred embodiment of this invention, the solid phase contains a lyophilised mixture of double-coated human milk enzymes, at least one sporified strain, glucans, carrageenans, colloidal silicon dioxide and a vitamin complex consisting of Niacin, calcium pantothenate, riboflavin, thiamine, Vitamin B6, Vitamin B12, Vitamin K and folic acid; the liquid phase contains fructose, honey, arabino galactooligosaccharides, flavourings, aloe vera, potassium sorbate, methyl paraben, propyl paraben, and sufficient demineralised water to make it up to the required volume. Said solution is acidified to pH 4.5 with a buffer solution of trisodium citrate.

The composition to which the invention relates is preferably administered with a vial on which a reservoir cap containing the solid phase is positioned, while the liquid phase is contained in the vial.

As clinical tests are extremely time-consuming and onerous to perform, the Applicant has evaluated the composition according to the invention, and specifically its protective thickening, by laboratory tests. In fact at the time of insertion of the solid phase into the liquid phase of the composition, a thickening is observed at a pH of 4.5 which increases as the pH declines, until the viscosity, measured with a high-resolution viscosimeter, reaches a value of up to 130 cps, and remains at that level until the pH returns from 5 to 5.5. This demonstrates that the composition according to the invention presents a protective thickening between pH 4.5 and 5.5.

According to a further preferred aspect of this invention, said composition should be administered after meals to increase the protective thickening effect, which enhances the vitality of the milk enzymes.

The pH in the stomach is known to vary during the day, according to food intake. The pH is 1-2 during the night, when the stomach is empty. The pH increases to 5-6 immediately after breakfast, and gradually declines to 1-3 when the stomach contents gradually enter the small intestine. The pH of the stomach contents is strongly influenced by the amount of food eaten and the period for which it remains in the stomach ("Intestinal Bacteria and Health", Tomotari Mitsuoka).

The composition according to the present invention is prepared by known techniques, and the following example illustrates the invention without limiting it in any way.

### EXAMPLE

### Preparation of the solid phase of the composition

Place the following materials in a suitable mixer with a mobile steel body after sieving through a 600 µm mesh screen:

| | |
|---|---|
| Mixture of double-coated human milk enzymes at 320 billion/g | 23,125.000 g |
| *Lactobacillus sporogenes* at 200 billion/g | 312.500 g |
| Glucans | 312.500 g |
| Carrageenans | 1,250.000 g |
| Colloidal silicon dioxide | 187.500 g |
| Vitamin complex 10.120 mg: | |
| Niacin | 405.000 g |
| Calcium pantothenate | 151.250 g |
| Riboflavin | 36.250 g |
| Thiamine | 31.500 g |
| Vitamin B6 | 37.500 g |
| 0.1% vitamin B12 | 22.500 g |
| Folic acid | 3.750 g |
| Vitamin K | 1.310 g |
| | 24.820 kg |

and mixing for 20 minutes.

The homogenous mixture is then discharged from the mixer into suitable plastic containers fitted with a double polythene bag which is sealed after filling, and a sample is sent for analysis.

### Preparation of solvent phase

Pour 100 litres of demineralised water into a stainless steel melter with a capacity of 600 litres, equipped with a propeller-operated agitation system, and heat to 50°C.

Pour 50 litres of demineralised water into a stainless steel container of suitable dimensions, and bring to the boil. Dissolve:

| | |
|---|---|
| potassium sorbate | 0.500 kg |
| methyl paraben | 0.375 kg |
| propyl paraben | 0.125 kg |

The solution containing the preservatives is combined with water in the melter, and 149 kg of fructose and honey is added under agitation; the temperature is increased to 80°C and agitation is continued overnight. 31.250 kg of arabino galactooligosaccharides, 0.625 kg of aloe vera and 0.700 kg of sour cherry flavouring are then added at the temperature of about 30°C under agitation, and the solution obtained is buffered to pH 4.5 with trisodium citrate and made up to a final volume of 500 litres with osmotised water.

## Claims

1. A nutraceutical composition **characterised in that** it contains probiotic bacteria with aloe and carrageenans, and other prebiotic substances.

2. Nutraceutical composition of claim 1, wherein the probiotic bacteria are at least 12 strains of double-coated lyophilised live human milk enzymes and at least one sporified strain of live milk enzymes.

3. Nutraceutical composition of claims 1 and 2, wherein the double-coated lyophilised live human milk enzymes are present in the quantity of at least 320 billion/g and the sporified strain of live milk enzymes is present in the quantity of at least 200 billion/g.

4. Nutraceutical composition of claims 1 to 3, containing 5 to 10 mg of aloe and 15 to 30 mg of carrageenans.

5. Nutraceutical composition of claims 1 to 4, containing lota carrageenan as carrageenans.

6. Nutraceutical composition of claims 1 to 5, containing arabino galactan, a fibre of the larch tree, as prebiotic substance.

7. Nutraceutical composition of claims 1 to 6 for oral use containing, in a solid phase, a lyophilised mixture of bi-coated human milk enzymes, at least one sporified strain, glucans, carrageenans, colloidal silicon dioxide and a vitamin complex consisting of Niacin, calcium pantothenate, riboflavin, thiamine, Vitamin B6, Vitamin B12, Folic Acid and Vitamin K; and, in a liquid phase, fructose, honey, arabino galactooligosaccharides, flavourings, aloe vera, potassium sorbate, methyl paraben, propyl paraben, and sufficient demineralised water to make it up to the required volume.

8. Use of a composition of claims 1 to 7 to prepare a composition with synbiotic activity.

9. Use according claim 8 of a composition suitable for administration after meals.

## Patentansprüche

1. Eine medizinisch wirksame Lebensmittelmischung, **gekennzeichnet dadurch, dass** sie probiotische Bakterien, Aloe und Carrageenans sowie andere präbiotische Substanzen enthält.

2. Eine medizinisch wirksame Lebensmittelmischung nach Anspruch 1, wobei die probiotischen Bakterien aus mindestens 12 Stämmen lyophilisierter lebender menschlicher Milchenzyme mit doppelter Umhüllung und mindestens einem Sporen-Stamm lebender Milchenzyme bestehen.

3. Eine medizinisch wirksame Lebensmittelmischung nach Anspruch 1 und 2, wobei die lyophilisierten lebenden menschlichen Milchenzyme mit doppelter Umhüllung in einer Menge von mindestens 320 Milliarden/g und der Sporen-Stamm lebender Milchenzyme in einer Menge von mindestens 200 Milliarden/g vorhanden sind.

4. Eine medizinisch wirksame Lebensmittelmischung nach den Ansprüchen 1 bis 3, die 5 bis 10 mg Aloe und 15 bis 30 mg Carrageenans enthält.

5. Eine medizinisch wirksame Lebensmittelmischung nach den Ansprüchen 1 bis 4, die als Carrageenans Jota Carrageenan enthält.

6. Eine medizinisch wirksame Lebensmittelmischung nach den Ansprüchen 1 bis 5, die Arabino-Galactan, eine Lärchenfaser, als präbiotische Substanz enthält.

7. Eine medizinisch wirksame Lebensmittelmischung nach den Ansprüchen 1 bis 6 zur oralen Einnahme, die in der festen Zubereitung eine lyophilisierte Mischung lebender menschlicher Milchenzyme mit doppelter Umhüllung, mindestens einen Sporen-Stamm, Glucan, Carrageenans kolloidales Siliciumdioxid und einen Vitaminkomplex enthält, der aus Niacin, Calcium-Pantothenate, Riboflavin, Thiamin, Vitamin B6, Vitamin B12, Folsäure und Vitamin K besteht; und in einer flüssigen Zubereitung Fruktose, Honig, Arabino-Galactooligosaccharide, Aromastoffe, Aloe Vera, Kaliumsorbat, Methylparaben, Propylparaben und genügend demineralisiertes Wasser, um das erforderliche Volumen zu erreichen, enthält.

8. Anwendung einer Mischung nach den Ansprüchen 1 bis 7 zur Vorbereitung einer Mischung mit symbiotischer Wirkung.

9. Anwendung nach dem Anspruch 8 einer Mischung, die zur Einnahme nach den Mahlzeiten geeignet ist.

## Revendications

1. Composition nutraceutique **caractérisée par** la présence de bactéries probiotiques contenant de l'aloe et du carragène, ainsi que d'autre substances prébiotiques.

2. Composition nutraceutique selon la revendication 1, où les probiotiques sont constitués d'au moins 12 souches de ferments lactiques humains vivants lyophilisés bi-couche et d'au moins une souche sporique de ferments lactiques vivants.

3. Composition nutraceutique selon l'une des revendications 1 à 2, dans laquelle on dénombre au moins 320 milliards/g de ferments lactiques humains vivants bi-couche et au moins 200 milliards/g de ferments lactiques de souche sporique.

4. Composition nutraceutique selon l'une des revendications 1 à 3, contenant 510 mg d'aloe et 15 à 30 mg de carragène.

5. Composition nutraceutique selon l'une des revendications 1 à 4, contenant du carragène Iota en tant que carragène.

6. Composition nutraceutique selon l'une des revendications 1 à 5, contenant du galactane arabique, une fibre de mélèze, en tant que substance prébiotique.

7. Composition nutraceutique selon les revendications de 1 à 6 à usage oral, contenant, dans sa phase solide, un mélange lyophilisé de ferments lactiques humains bi-couche, au moins une souche sporique, des glucanes, du carragène, du dioxyde de silicone colloïdal et un complexe vitaminé constitué de niacine, calcium panthoténate, riboflavine, thiamine, vitamine B6, vitamine B12, acide folique et vitamine K, et, dans sa phase liquide, du fructose, du miel, des galactooligosaccharides arabiques, des arômes, de l'aloe vera, du sorbate de potassium, du méthylparaben, du probylparaben et suffisamment d'eau déminéralisée pour obtenir le volume nécessaire.

8. L'utilisation d'une composition selon l'une des revendications de 1 à 7 pour préparer une composition à activité symbiotique.

9. L'utilisation selon la revendication 8 d'une composition pouvant être administrée après les repas.
